# EUROPÄISCHE PATENTSCHRIFT

(11) **EP 2 083 679 B1**
(45) Veröffentlichungstag und Bekanntmachung des Hinweises auf die Patenterteilung: **22.12.2010**
(21) Anmeldenummer: 07819199.6
(22) Anmeldetag: 22.10.2007
(51) Int. Cl.: A61B 5/00, A61B 18/00

(54) **MEDIZINISCHES INSTRUMENT, DAS EINEN KLEBSTOFFVERGUSS MIT FÜLLSTOFF ENTHÄLT**
MEDICAL INSTRUMENT CONTAINING AN ADHESIVE SEAL WITH FILLER
INSTRUMENT MÉDICAL QUI CONTIENT UNE PÂTE ADHÉSIVE DOTÉE D'UNE CHARGE

(30) Priorität: 23.10.2006 DE 102006050969
(43) Veröffentlichungstag der Anmeldung: 05.08.2009
(73) Patentinhaber: Karl Storz GmbH & Co. KG, 78532 Tuttlingen (DE)
(72) Erfinder: HUBER, Matthias, 78576 Emmingen-Liptingen (DE); EISENKOLB, Peter, 78532 Tuttlingen (DE)
(74) Vertreter: Weller, Wolfgang
(86) Internationale Anmeldenummer: PCT/EP2007/009135
(87) Internationale Veröffentlichungsnummer: WO 2008/049563

(56) Entgegenhaltungen:
- EP-A- 1 847 213
- GB-A- 1 122 672
- JP-A- 2002 238 834
- JP-A- 2003 126 023
- US-A1- 2005 288 554

## Beschreibung

Die Erfindung betrifft ein Verfahren zum Herstellen eines autoklavierbaren medizinischen Instruments, bei dem Klebstoff, der einen Füllstoff in Form von Partikeln enthält, zum Füllen eines Hohlraumes vergossen wird, sowie ein daraus resultierendes medizinisches Instrument.

Bei der Herstellung von medizinischen Instrumenten ist es gebräuchlich, beim Montieren des Instruments gewisse Bauteile über einen Klebstoffverguss miteinander zu verbinden bzw. einzubetten. Der Klebstoffverguss hat zum einen den Sinn, die Bauteile untereinander zu positionieren und zu fixieren, was durch die ausgehärtete Klebstoffmasse erfolgt. Ein weiterer wesentlicher Punkt ist, für eine dichtende Verbindung zwischen diesen Bauteilen zu sorgen, um zu verhindern, dass von der Außenseite her Kontaminationen in das Innere des Instrumentes eindringen können. Solche Kontaminationen können Körper- oder Gewebeflüssigkeiten sein, mit denen das medizinische Instrument während des chirurgischen Einsatzes in Kontakt tritt. Kontaminationen können aber auch gasförmige oder flüssige Reinigungsmittel sein, die bei den nach den medizinischen Eingriffen durchgeführten Reinigungs- und Sterilisierungsvorgängen angewendet werden. Dabei kann es sich um sehr aggressive Reinigungsmittel handeln, beispielsweise Peroxyd enthaltende Reinigungsmittel. Darüber hinaus werden beim Autoklavieren, insbesondere beim so genannten Blitzautoklavieren, die Instrumente auf Temperaturen von über 145 °C erhitzt und sehr rasch wieder abgekühlt.

Im praktischen Einsatz wurde nun festgestellt, dass diese extremen Belastungen, die auf ein chirurgisches Instrument wiederholt einwirken, beispielsweise ein Endoskop, das häufig eingesetzt wird, nach und nach zu Rissen in dem Klebstoffverguss führen.

Dies resultiert nicht nur aus den extremen thermischen Belastungen beim Autoklavieren selbst, sondern Rissbildungen werden noch dadurch gefördert, dass der Klebstoff, der ja aus einem ausgehärteten Kunststoffmaterial besteht, meist einen Raum zwischen metallischen Materialien ausfüllt, meist Medizinerstahl. Diese Materialien weisen andere thermische Eigenschaften auf als der Klebstoff als solcher. Somit können nicht nur in dem Klebstoff selbst Risse entstehen, sondern der Klebstoff löst sich von der Grenzübergangsfläche zum meist metallischen Material, das mit dem Klebstoff vergossen ist, teilweise ab.

Somit können im Langzeitbetrieb durch diese Risse nach und nach Kontaminationen von außen in das Innere des medizinischen Instruments eindringen. Diese beeinträchtigen die Funktionssicherheit des Instrumentes.

Aus der britischen Patentschrift GB 1,122,672 ist ein medizinisches Instrument in Form eines dentalen Mundspiegels bekannt. Eine distale Befestigungsplatte dient dazu, dass daran der Spiegel mittels eines Klebstoffes befestigt wird. Der Klebstoff ist mit einem Metallpulver, z.B. Eisenpulver, Aluminiumpulver und dergleichen, vermischt. Dadurch wird die Wärmeleitfähigkeit des Klebstoffes deutlich erhöht. Damit können thermische Spannungen abgebaut werden, so dass der Mundspiegel zahlreichen Sterilisiervorgängen bei erhöhten Temperaturen widersteht, ohne dass sich die Klebeverbindung zwischen Befestigungsplatte und Spiegel löst. Der Anteil des zugemischten Pulvers wird durch die Zusammensetzung des Klebstoffes, der Schichtdicke der Klebschicht und der gewünschten Wärmeleitfähigkeit beeinflusst, ohne dass weitere Angaben über Korngrößenverteilung oder Volumenteile gegeben sind.

Aus US2005/0288554 ist bekannt dem Endoskopmaterial feine Metallpartikel zuzumischen, um es Röntgen-positiv zu machen.

Aus JP-A-2002 238834 ist bekannt einem Klebstoff bis zu 50 Gewichtsprozent Gummi- oder Plastikanteile zuzumengen um die Beständigkeit beim Autoklavieren zu erhöhen.

Es ist Aufgabe der vorliegenden Erfindung, ein Verfahren zum Herstellen eines medizinischen Instruments und ein entsprechendes medizinisches Instrument zu schaffen, das einen Klebstoffverguss aufweist, der auch nach zahlreichen Autoklaviervorgängen dicht ist, so dass keine Kontaminationen über den Klebstoffverguss in das Innere des Instrumentes eindringen können.

Erfindungsgemäß wird bei dem Verfahren die Aufgabe dadurch gelöst, dass der Klebstoff in dünnflüssiger Form vergossen wird, dass die Partikel des Füllstoffs eine Dichte aufweisen, die sich von der Dichte des dünnflüssigen Klebstoffes unterscheiden, und dass den Partikeln des Füllstoffes eine ausreichende Zeitspanne zur Verfügung gestellt wird, um sich zu einer möglichst dichten Partikelpackung, die entsprechend ihrer Geometric möglich ist, zusammenzulegen, und dass erst dann der Klebstoff aushärtet.

Diese Maßnahmen haben nun zahlreiche erhebliche Vorteile.

Durch Verarbeitung des Klebstoffes in dünnflüssiger Form kann dieser auch in dünnste Hohlräume, Spalte oder dergleichen vergossen werden, ohne dass sich Luftblasen oder sonstige Einschlüsse bilden. Dadurch, dass die Partikel eine Dichte aufweisen, die sich von der Dichte des dünnflüssigen Klebstoffes unterscheidet, erfolgt eine Art Aufschlämmen oder ein Aufschwimmen der Partikel im dünnflüssigen Klebstoff, je nachdem, ob die Dichte größer oder kleiner ist. Aufgrund der Bereitstellung einer gewissen Zeitspanne ist es den Partikeln nunmehr möglich, sich zu einer möglichst dichten Partikelpackung zusammenzulegen.

Nimmt man das Beispiel, bei dem die Partikel eine höhere Dichte aufweisen als der dünnflüssige Klebstoff, tendieren diese ja dazu, sich in Schwerkraftrichtung zu bewegen, also in einer Flüssigkeit abzusinken. Beim Einbringen der Aufschlämmung sind diese zunächst statistisch mehr oder weniger verteilt und werden durch den Einbringvorgang verwirbelt. Nach dem Einbringen setzen sich die Partikel ab, und zwar zu einer möglichst dichten Partikelpackung bzw. der dichtesten, die entsprechend der Geometrie möglich ist. Dies kann noch bspw. durch Klopfen oder durch Vibrieren der Vorrichtung unterstützt werden.

Nimmt man das Beispiel sphärischer Partikel, also Kugeln, so ist es möglich, die Partikel nahe der idealen dichtesten Kugelpackung zusammenzulegen, wobei in diesem Fall der Raumerfüllungsgrad einer dichtesten Kugelpackung 74 % beträgt. Nur die Zwischenräume zwischen den Partikeln und die Räume zwischen den Partikeln und der Wand des Hohlraumes sind mit dem Klebstoff gefüllt. Es ist möglich so zu verfahren, dass der gesamte Bereich des Hohlraumes derart aufgefüllt wird. Man füllt den dünnflüssigen Klebstoff in den Hohlraum ein, lässt den Partikeln eine gewisse Zeit, um sich abzusetzen und füllt dann weiteren Klebstoff nach, aus dem sich dann weitere Partikel auf die schon bereits abgesetzten Partikel absetzen, so dass nach und nach der gesamte Hohlraum mit der gewünschten dichten Partikelpackung aufgefüllt werden kann.

Dies muss nicht vollständig erfolgen, sondern kann auch nur bereichsweise erfolgen, bspw. wenn es ausreichend ist, dass dies nur in einem besonders kritischen Bereich erfolgt, z.B. in dem sich hohe thermische Spannungen aufbauen können und in dem die Gefahr besteht, dass von der Außenseite her Kontaminationen eindringen.

Anschließend wird der Klebstoff ausgehärtet.

Dadurch entsteht ein Verbundmaterial, das in hohem Maße volumenmäßig durch die Partikel belegt ist.

Durch entsprechende Auswahl des Materials der Partikel können dem Verbundmaterial die nachgesuchten Eigenschaften verliehen werden, nämlich dass dieses Verbundmaterial die thermischen und die mechanischen Belastungen auf Dauer erträgt. Die Partikel verleihen dem Verbundmaterial elektrisch leitende oder isolierende Eigenschaften, je nachdem, wie das Material der Partikel ausgewählt ist. Der Fachmann hat eine Vielzahl von Partikelmaterialien zur Verfügung, die es ihm in Abhängigkeit von den Materialien, die den Hohlraum umgrenzen, erlauben, ein Verbundmaterial zu kreieren, das optimal auf diese Materialien abgestimmt ist, so dass optimale Wärmebrücken entstehen, wodurch innere Spannungen im Verbundmaterial abgebaut werden können.

Im medizinischen Bereich bestehen die Bauteile meist aus Metall, insbesondere aus Medizinerstahl, so dass die Partikel bspw. in Form eines entsprechenden Stahlpulvers ausgewählt werden können, so dass möglichst geringe thermische Spannungen entstehen. Durch die Möglichkeit, das Verbundmaterial mit einem sehr hohen Volumenanteil an Partikeln aufzufüllen, entsteht ein Verbundmaterial, das eher einem metallischen Sintermaterial ähnelt als einem Klebstoff. Dieses Verbundmaterial weist eine hohe Wärmeleitfähigkeit auf, so dass die Ausbildung von thermischen Spannungen weitgehend unterbunden werden kann.

Wie zuvor erwähnt, ist es auch möglich, Partikel einzuarbeiten, die eine geringere Dichte als der flüssige Kunststoff aufweisen, bspw. ein Füllstoff aus Glasmaterialien oder Kunststoffmaterialien. Diese werden dann auf der Oberfläche des dünnflüssigen Klebstoffes aufschwimmen.

In diesem Fall wird man das Aufstapeln oder das Zusammenlegen der Partikelpackung nicht durch kontinuierliches Absinken von zusätzlichen Partikeln erzielen, sondern umgekehrt, durch immer mehr Aufschwimmen von solchen Materialien. Man füllt den Hohlraum also nicht durch Aufstapeln von unten nach oben, sondern umgekehrt durch Aufstapeln von oben nach unten.

Die Verfahrensweise ermöglicht dem Fachmann somit, auch auf unterschiedlichste Materialien zu reagieren, die mit dem Klebstoff vergossen werden sollen.

In einer weiteren Ausgestaltung der Erfindung wird das Material der Partikel derart ausgewählt, dass deren thermische Eigenschaften nahe der thermischen Eigenschaften der zu vergießenden Materialien des medizinischen Instruments liegen und dadurch gewünschte mechanische sowie elektrische Eigenschaften erfüllt werden.

Zuvor wurde ja schon erwähnt, dass im medizinischen Bereich metallische Materialien eingesetzt werden, so dass es sich anbietet, entsprechende metallische Materialien als Füllstoff einzusetzen. Darauf ist aber die Lehre nicht beschränkt, es können auch andere Materialien herangezogen werden, sofern sie eben den thermischen Eigenschaften der zu vergießenden Bauteile möglichst nahe kommen bzw. gewünschte mechanische sowie elektrische Eigenschaften erfüllen.

Es ist auch möglich, beide Arten von Partikeln, also mit unterschiedlichen Dichten, in den dünnflüssigen Klebstoff einzuarbeiten, und zwar solche, die aufschwimmen, als auch solche, die absinken, wenn bspw. bei der Verbindung von zwei ineinander geschobenen Schäften an einem Ende bspw. Kunststoff oder Glasmaterialien angebracht sind und am gegenüberliegenden Ende im Wesentlichen nur metallische Materialien den Hohlraum begrenzen. Dadurch entsteht ein Verbundmaterial, das an einer Seite, in Schwerkraftrichtung gesehen oben, überwiegend eine Partikelpackung mit den leichteren Teilen bspw. Kunststoff oder Glas enthält, wenn es aus Gründen der elektrischen oder Wärmeleitfähigkeit bzw. der mechanischen Festigkeit oder Dämpfung günstig ist, solche Materialien im Verbundmaterial zu haben, wohingegen am gegenüberliegenden Ende sich dann bspw. die schweren metallischen Materialien abgesetzt haben.

In einer weiteren Ausgestaltung der Erfindung ist die Größe und die Form der Partikel so ausgewählt, dass zwischen den zusammengelegten Partikeln Freiräume einer solchen geringen Größe vorhanden sind, dass der in den Freiräumen ausgehärtete Klebstoff den thermischen Belastungen beim Autoklavieren auf Dauer widersteht.

Diese Maßnahme beruht auf den folgenden Überlegungen. Dünnflüssige Klebstoffe härten meist zu relativ spröden, harten, glasartigen Materialien aus. Dennoch weisen diese bis zu einer gewissen Materialdicke thermische Eigenschaften auf, die auch den thermischen Belastungen beim Autoklavieren widerstehen können. Erst bei relativ kompakten oder relativ großen Schichtdicken sind Rissbildungen und ggf. Materialausbrüche festzustellen. Von den Herstellern solcher Klebemittel werden die Daten über diese Schichtdicken verbreitet.

Die Lehre dieser Ausgestaltung beinhaltet nunmehr, die Form und Größe der Partikel so auszuwählen, dass in den Zwischenräumen zwischen den Partikeln nur solche Mengen an ausgehärtetem Kunststoff aufgenommen werden können, die noch den Spezifikationen bezüglich der thermischen Widerstandsfähigkeit entsprechen.

Nimmt man wieder das Beispiel der kugelförmigen Partikel heran, so kann man errechnen, wie groß der maximale Abstand in der dichtesten Kugelpackung zwischen den Kugeln einer bestimmten Größe ist. Wenn dieser maximale Abstand der Spezifikation des Klebstoffes entspricht, also in dieser Materialstärke den thermischen Belastungen beim Autoklavieren widerstehen kann, wird der Fachmann eine solche Größe aussuchen, dass diese Bedingungen des maximalen Abstandes erfüllt werden.

Entsprechendes gilt natürlich auch für Füllstoffe, die aus anderen geometrischen Körpern als aus Kugeln bestehen.

Will man die Freiräume nicht ausschließlich mit Klebstoff füllen, kann man eine Mischung aus Partikeln auswählen, die relative große und kleine Partikel aufweist, wobei die kleinen so ausgewählt sind, dass diese in die Freiräume zwischen den großen Partikeln passen.

In einer weiteren Ausgestaltung der Erfindung werden Partikel im Bereich von 50 bis 150 µm eingesetzt.

Diese Partikelgröße hat sich im Instrumentenbau als sehr vorteilhaft erwiesen, denn mit diesen Partikelgrößen können die üblicherweise zu vergießenden Freiräume oder Spalte optimal gefüllt bzw. vergossen werden.

In einer weiteren Ausgestaltung der Erfindung besteht der Füllstoff aus metallischem Material, aus Glasperlen, Karbid-, Kunststoff- oder Keramikteilchen. Es kann also nach Wahl um elektrisch leitende und/oder wärmeleitende sowie um elektrisch isolierende und/oder wärmeisolierende Eigenschaften nachgesucht werden.

Der Füllstoff kann aus sphärischen, scheibenförmigen, ringförmigen, kubischen oder vielflächigen Partikeln, also Formstücken bestehen.

Diese Auswahl an Materialien und geometrischen Formen erlaubt dem Fachmann, unterschiedliche Verbundmaterialien zu schaffen, die jeweils optimal auf den Einsatzfall anzupassen sind.

In einer weiteren Ausgestaltung der Erfindung liegt der Volumenanteil an Partikeln im Klebstoff, vor der Aushärtung, im Bereich von 20 bis 80 %.

Es hat sich gezeigt, dass in diesen Volumenbereichen das dünnflüssige Klebematerial mit den darin eingearbeiteten Partikelteilchen sich besonders günstig verarbeiten lässt. Im Betrieb sowie bei der Reinigung und Sterilisierung zeigen solche medizinischen Instrumente hervorragende Eigenschaften.

In einer weiteren Ausgestaltung der Erfindung wird das Verfahren so durchgeführt, dass im endfertigen ausgehärteten Kunststoff der Volumenanteil der Partikel 50 bis 80 % beträgt.

Diese Maßnahme hat den Vorteil, dass ein Verbundmaterial entsteht, dessen Volumen im Wesentlichen durch die Eigenschaften des Füllmaterials und nicht durch die Eigenschaften des Klebstoffes bestimmt wird, so dass neben der Notwendigkeit, die einzelnen Partikel durch einen Klebstoff miteinander zu verbinden, um überhaupt den Verbund auszubilden, die Eigenschaft im Wesentlichen durch das Füllmaterial bestimmt wird.

Die zwei Hauptaufgaben des Verbundmaterials sind, zum einen eine möglichst dichte Haftung im Sinne einer Halterung oder eine Klebeverbindung von zwei aneinander liegenden Teilen zu bewerkstelligen und zum anderen einen auf Dauer dichten Verguss zu bilden, wovon die zweite Hauptaufgabe vordergründig von der Erfindung gelöst wird, ohne der ersten Hauptaufgabe weniger Rechnung zu tragen. Die zu vergießenden Bauteile sind ohnehin bei einem Instrument durch anderweitige technische Maßnahmen relativ zueinander gehalten.

Aufgrund der Tatsache, dass der resultierende Klebstoffverguss den extremen thermischen Schwankungen beim Autoklavieren im medizinischen Bereich gut widerstehen kann, eröffnet sich die Möglichkeit, Formteile in den Füllstoff enthaltenen Klebstoff einzugießen. So können bspw. sehr kompliziert geformte Kanäle, die durch eine spanende Bearbeitung nicht oder nur sehr aufwändig hergestellt werden können, dadurch hergestellt werden, dass man solche Kanäle direkt in dem Klebstoff ausbildet. Dies kann bspw. so erfolgen, dass durch Formteile, bspw. gewundene Drähte oder Schnüre, in dem zu vergießenden Hohlraum die Form der Kanäle vorgegeben wird, anschließend dieser Hohlraum mit dem Kunststoffmaterial ausgegossen wird, und nach Aushärtung die Formteile, also die Schnüre oder Drähte, ausgebracht, z.B. ausgeschmolzen, ausgebrannt, ausgeklopft oder wie beim Entfernen des Kerns beim Eisengießen entfernt werden. Die Schnüre bzw. Drähte können aber auch in der Vergussmasse verbleiben. Es können z.B. faser- oder kanalartige Füllstoffkörper in den Vergussraum eingebracht werden, der dann mit dem Klebstoff ausgefüllt wird, so dass zwischen den Füllkörpern jeweils nur ein geringer Klebstoffspalt verbleibt und somit nur geringe Rissneigung bzw. Rissfortschreitneigung besteht. So könnte sich bspw. durch eine Vielzahl von solchen Füllstoffkanälen eine Art Wabenstruktur ergeben.

Dies ist deswegen möglich, da nun auch relativ großvolumige Bereiche an Klebstoffverguss hergestellt werden können, da diese, wie zuvor erwähnt, die extremen thermischen Belastungen ohne Rissbildung aufnehmen können. Dies eröffnet beachtliche Möglichkeiten, im Instrumentenbau die Materialien Medizinerstahl und Klebstoff in Kombination auch unter Ausbildung relativ volumengroßer Klebstoffvergussbereiche einzusetzen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand der beiliegenden Zeichnungen näher beschrieben und erläutert. Es zeigen:
- Fig. 1: teilweise geschnitten eine Seitenansicht eines medizinischen Instru- ments mit einem erfindungsgemäßen Kunststoffverguss,
- Fig. 2: eine stark vergrößerte Seitenansicht des Instrumentes von Fig. 1, und
- Fig. 3: einen weiter stark vergrößerten Ausschnitt eines medizinischen In- strumentes, in dem ein Hohlraum mit dem erfindungsgemäßen Ver- bundmaterial vergossen worden ist.

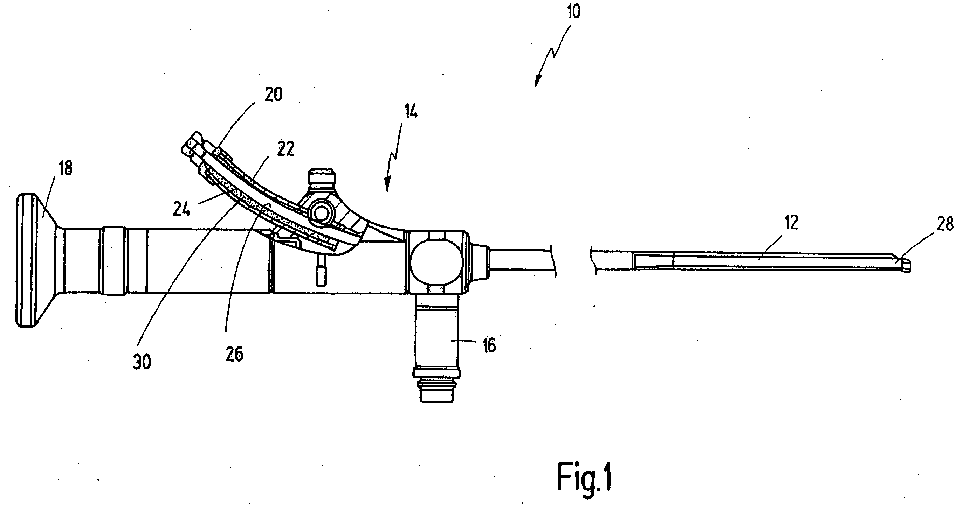

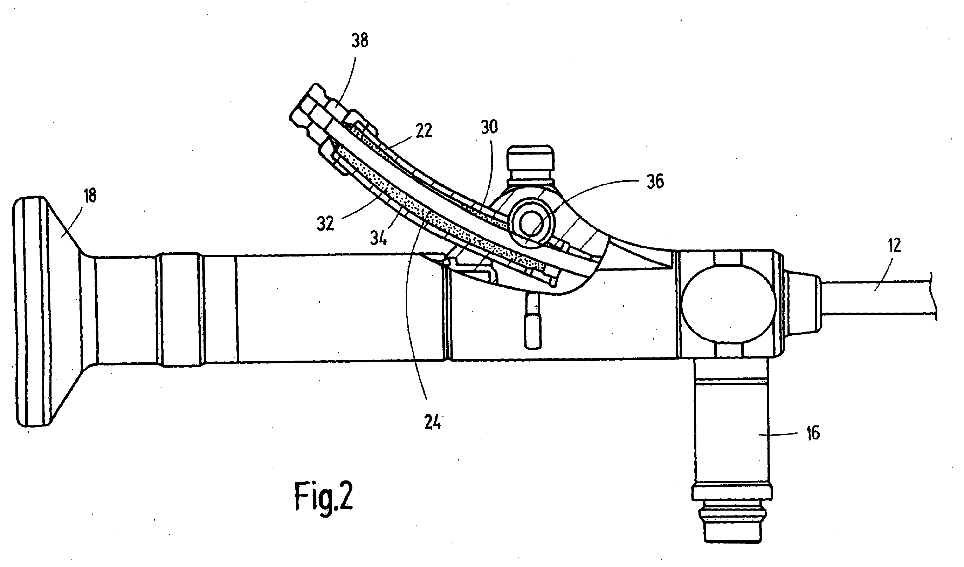

Ein in den Fig. 1 und 2 dargestelltes medizinisches Instrument ist in seiner Gesamtheit mit der Bezugsziffer 10 bezeichnet.

Bei dem medizinischen Instrument handelt es sich um ein Uretero-Renoskop bzw. ein Ureteroskop.

Das Instrument 10 weist einen Schaft 12 auf, dessen proximales Ende in einem Gehäuse 14 aufgenommen ist.

Vom Gehäuse 14 steht seitlich ein Lichtleiteranschluss 16 vor. Proximalseitig ist das Gehäuse mit einem Okular 18 versehen.

Im Schaft 12 ist eine hier nicht näher dargestellte Optik aufgenommen, so dass über das Okular durch den Schaft 12 hindurch beobachtet werden kann. Über den seitlichen Lichtleiteranschluss 16 wird Licht in einem ebenfalls im Schaft geführten Lichtleiter zum distalen Ende 28 des Schaftes geführt. Der Schaft 12 ist relativ dünn, so dass er beispielsweise in eine Harnröhre eingeführt werden kann.

Vom Gehäuse steht seitlich, bogenartig gekrümmt, ein Anschluss 20 vor. Über den gekrümmten Anschluss 20 kann seitlich in den Schaft ein Arbeitsinstrument eingeführt werden, mit dem entweder im Harnleiter selbst oder in der Blase Manipulationen vorgenommen werden können.

Wie aus der Schnittdarstellung ersichtlich, besteht der gekrümmte Anschluss 20 aus einem äußeren Verstärkungsrohr 22, in dem ein Innenrohr 24 aufgenommen ist. Das Innenrohr 24 umgrenzt den eigentlichen Arbeitskanal 26, über den das Arbeitsinstrument in einen inneren, hier nicht näher dargestellten Arbeitskanal im Schaft 12 eingeschoben werden kann.

Das heißt, das Innenrohr 24 geht in einen geradlinigen Abschnitt über, der am distalen Ende 28 des Schaftes 12 mündet. Aus der Schnittdarstellung ist zu erkennen, dass die Krümmungen des äußeren Verstärkungsrohrs 22 und des Innenrohrs 24 unterschiedlich sind. Zwischen der Außenseite des Innenrohrs 24 und der Innenseite des Verstärkungsrohrs 22 ist somit ein Zwischenraum 30 vorhanden, der mit einem Klebstoff 32, der einen Füllstoff 34 enthält, vergossen wird. Dadurch wird nicht nur eine Lagefixierung bewerkstelligt, sondern es wird auch der Zwischenraum 30 zwischen Innenrohr 24 und Verstärkungsrohr 22 zur Außenseite hin abgedichtet.

Aus der Schnittdarstellung von Fig. 2 ist zu erkennen, dass in den Arbeitskanal 22 noch ein Queranschluss 36 mündet, um beispielsweise durch den Arbeitskanal 26, neben dem Arbeitsinstrument auch Saug- oder Spülflüssigkeiten zuführen zu können. Wie insbesondere aus der vergrößerten Darstellung von Fig. 2 zu erkennen, schneidet der Queranschluss 36 die Vergussmasse.

In den Klebstoff 32 wird bei erhöhter Temperatur (50 - 70 °C), also im dünnflüssigen Zustand, ein Füllstoff 34 eingearbeitet, der aus sphärischen Metallpartikeln besteht, die eine Partikelgröße im Bereich von 50 bis 150 µm aufweisen. In den wasserdünnen erwärmten Klebstoff 32 werden etwa 20 bis 80 Vol.-% des Füllstoffes 34 eingetragen.

Die Partikel können auch aus scheiben- oder ringförmigen, kubischen bzw. allgemein vielflächigen Partikeln, z.B. Flakes, bestehen.

Bei der Montage des Instrumentes 10 kann so vorgegangen werden, dass sowohl das Verstärkungsrohr 22 als auch das Innenrohr 24 im Gehäuse 14 montiert werden. Eine endseitige Kappe 38 ist zu diesem Zeitpunkt noch nicht aufgesetzt, so dass der Zwischenraum 30 zwischen dem Innenrohr 24 und dem Verstärkungsrohr 22 von der Außenseite her zugänglich ist. Über diese Außenseite kann das erwärmte Gemisch aus Klebstoff 32 und Füllstoff 34 eingebracht werden, und der Zwischenraum 30 wird vollständig und insbesondere luftblasenfrei gefüllt. Durch entsprechendes Klopfen und Ausrichten des Anschlusses 20 in vertikaler Richtung kann der Austritt von Luft aus dem Zwischenraum noch begünstigt werden. Es ist auch möglich, kurzzeitig über ein Ultraschallgerät den Zusammenbau in entsprechende Schwingung zu versetzen.

Anschließend wird der Zusammenbau in einen Härteofen gegeben und dabei ausgehärtet.

Nach dem Aushärten des Klebstoffes wird die endseitige Fläche des Anschlusses bearbeitet, z.B. geschliffen, und die Kappe 38 wird aufgesetzt.

Anschließend kann noch durch Bohren der Queranschluss 36 bewerkstelligt werden.

Der Verbund zwischen Gussmasse und den metallischen Rohren 22 und 24 ist so innig, dass eine solche spanabhebende Bearbeitung (also Bohrung) durchgeführt werden kann, ohne Undichtigkeiten zu verursachen.

Es ist auch möglich, die Baugruppe des gekrümmten Anschlusses 20 als vom Gehäuse 14 abnehmbares Bauteil auszubilden und abseits vom Instrument 10 den Klebstoffverguss auszubilden, und anschließend diesen Zusammenbau am Gehäuse zu montieren.

Da, wie zuvor erwähnt, der Klebstoff 32 mit dem Füllstoff 34 einen Klebstoffverguss ergibt, der den harten Bedingungen bei dem Reinigen, beim Einsatz in der Chirurgie, beim Handhaben (z.B. bei einem versehentlichen Herabfallen) und insbesondere bei dem Autoklavieren von solchen Instrumenten widersteht, kann der Arbeitskanal 26 auch anders bewerkstelligt werden. Anstelle des Innenrohrs 24 könnte beispielsweise ein entsprechend ausgeformter Draht oder eine entsprechende Schnur gelegt werden, die nach dem Aushärten des Klebstoffes wieder abgezogen wird. Hier könnte ein Trennmittel bzw. ein Adhäsionsverhinderer auf den Draht bzw. die Schnur aufgetragen werden, um das Abziehen zu erleichtern. Das heißt, der Arbeitskanal 26 entsteht dann direkt in der ausgehärteten Masse des Klebstoffes 32 selbst, ohne dass dazu ein entsprechendes Innenrohr 24 notwendig ist. Mit anderen Worten ausgedrückt umgrenzt der ausgehärtete Klebstoff den Arbeitskanal 26 selbst. Dies wird dann eingesetzt werden, wenn besonders gekrümmte oder verschlungene Arbeitskanäle bewerkstelligt werden, die durch ein Innenrohr 24 oder eine sonstige spanende Bearbeitung nicht oder nur sehr aufwändig hergestellt werden können. Dies deswegen, da erfindungsgemäß mit dem Füllstoff ein Klebstoffverguss resultiert, der den harten Bedingungen, denen die medizinischen Instrumente bei der Handhabung und bei der anschließenden Reinigung und Autoklavierung ausgesetzt sind, widerstehen kann.

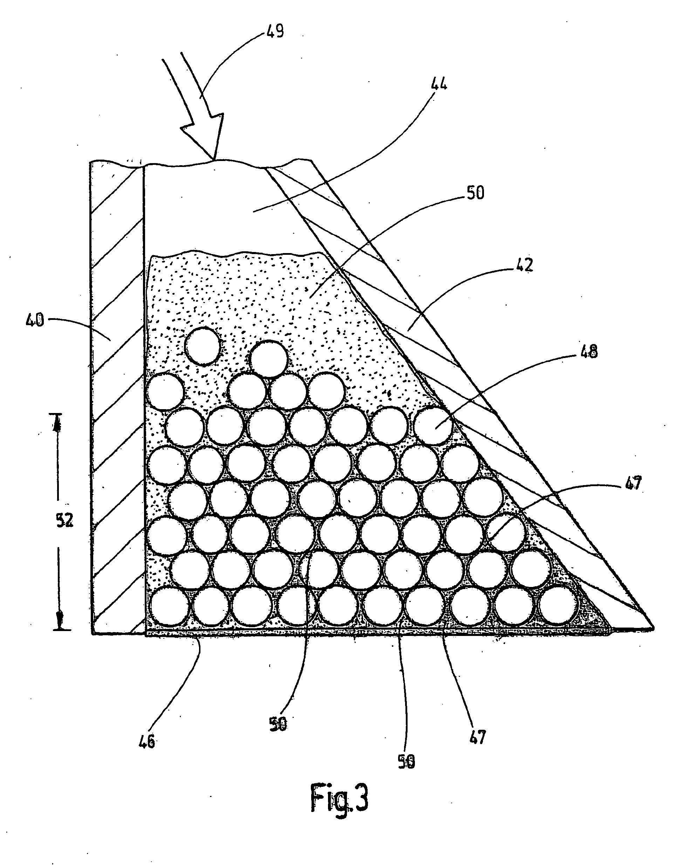

In Fig. 3 ist dargestellt, wie ein Hohlraum 44 zwischen zwei Bauteilen 40 und 42 mit dem erfindungsgemäßen Verfahren vergossen worden ist.

Der Hohlraum 44 zwischen den Bauteilen 40 und 42 wird durch einen Abschluss 46 zunächst abgeschlossen, wenn bspw. diese Seite die zur Außenseite hingewandte Seite ist.

In den Hohlraum 44 wird von "oben", wie das durch ein Pfeil 49 angedeutet ist, der erwärmte dünnflüssige Klebstoff, in dem Partikel 48 in Form von Kugeln eingemischt sind, eingefüllt. Die Kugeln bestehen aus einem metallischen Pulver, dessen Dichte höher ist als die Dichte des dünnflüssigen Klebstoffes. Die Dünnflüssigkeit des Klebstoffes liegt dabei im Bereich der Dünnflüssigkeit von Wasser. Den Partikeln 48 wird nun ein gewisser Zeitraum belassen, so dass sie sich der Schwerkraft folgend absenken und nach und nach zu einer dichten Kugelpackung zusammenlegen.

Dies kann, wie zuvor erwähnt, noch dadurch unterstützt werden, dass die Bauteile in Schwingungen versetzt werden.

Idealerweise legen sich die Partikel 48 zu einer dichtesten Kugelpackung zusammen. In den Freiräumen 47 zwischen den Partikeln 48 und auch zwischen den Partikeln 48 und den Bauteilen 40 und 42 verteilt sich gleichmäßig der noch dünnflüssige Klebstoff 50.

In Fig. 3 ist eine Situation dargestellt, bei der nur ein gewisser Bereich 52 des Hohlraumes 44 mit der dichtesten Kugelpackung belegt wird. Über dem Bereich 52 ist, nach einem gewissen Übergangsbereich mit mehr oder weniger regelmäßig verteilten Kugeln, ein Bereich vorhanden, der ausschließlich durch ausgehärteten Klebstoff 50 belegt ist.

Diese Verteilung kann gewünscht sein, wenn im Wesentlichen die beiden Bauteile 40 und 42 in Richtung der in der Darstellung von Fig. 3 unteren Außenseite hin abgedichtet werden sollen. Es ist dadurch ausgeschlossen, dass in dem Bereich 52 auch nach zahlreichen Autoklaviervorgängen Rissbildungen oder dergleichen entstehen, die ein Eindringen von Gasen oder Flüssigkeiten von außen ermöglichen.

Wenn in weiter innen liegenden Bereichen, in denen überwiegend ausgehärteter Klebstoff 50 vorhanden ist, Risse entstehen sollten, ist dies nicht weiter kritisch.

Sollte es aufgrund der konstruktiven Gegebenheiten aber dennoch kritisch sein, kann der Hohlraum 44 weiter mit dem Klebstoff/Partikelgemisch gefüllt werden, so dass sich weitere Partikel nach unten auf die bereits aufgeschichteten Partikel aufschichten.

Ursprünglich kann der Volumenanteil der Partikel im dünnflüssigen Klebstoff relativ gering angesetzt werden, um bspw. das Einbringen des Kunststoff/Partikelgemisches in einen sehr kleinen oder engen Hohlraum 44 zu erleichtern. Erst nach Absinken durch die Schwerkraft bildet sich nach und nach der Bereich 52 mit der kompakten dichtesten Kugelpackung aus.

Wenn gewünscht ist, den Hohlraum 44 vollständig zu bestücken, kann man entweder versuchen, schon in der ausgänglichen Mischung eine hohe Partikeldichte zu erreichen, was aber meist nicht die Handhabung fördert, oder man füllt so lange nach, bis der gesamte Hohlraum 44 mit der Partikelpackung belegt ist.

Nach dem gewünschten Befüllungszustand wird dann der Klebstoff ausgehärtet.

Wie zuvor erwähnt, besteht auch die Möglichkeit, mit Partikeln zu arbeiten, die aufschwimmen.

Hierzu muss man sich die Fig. 3 auf den Kopf gestellt vorstellen, so dass dann von unten mit dem Kunststoff befüllt wird, so dass nach und nach immer mehr Partikel aufschwimmen, die sich zu der kompakten dichtesten Kugelpackung zusammenlegen.

Der Abschluss 46 kann nach Ausbildung des Verbundes abgenommen werden oder, falls dies gewünscht ist, mit eingegossen werden.

## Patentansprüche

1. Verfahren zum Herstellen eines autoklavierbaren, medizinischen Instrumentes (10), bei dem ein Klebstoff (32), der einen Füllstoff (34) in Form von Partikeln (48) enthält, zum Füllen eines Hohlraumes (44) vergossen wird, **dadurch gekennzeichnet, dass** der Klebstoff (32, 50) in dünnflüssiger Form vergossen wird, dass die Partikel (48) des Füllstoffes (34) eine Dichte aufweisen, die sich von der Dichte des dünnflüssigen Klebstoffes unterscheidet, und dass den Partikeln (48) des Füllstoffes (34) eine ausreichende Zeitspanne zur Verfügung gestellt wird, um sich zumindest in einem Bereich (52) zu der dichtesten Partikelpackung, die entsprechend ihrer Geometrie möglich ist, zusammenzulegen, und dass erst dann der Klebstoff aushärtet.

2. Verfahren nach Anspruch 1, **dadurch gekennzeichnet, dass** das Material der Partikel (48) derart ausgewählt ist, dass deren thermische Eigenschaften nahe den thermischen Eigenschaften der zu vergießenden Bauteile (40, 42) des medizinischen Instrumentes liegen.

3. Verfahren nach Anspruch 1 oder 2, **dadurch gekennzeichnet, dass** die Größe und die Form der Partikel (48) so ausgewählt wird, dass zwischen den zusammengelegten Partikeln Freiräume (47) einer solchen geringen Größe vorhanden sind, dass der in den Freiräumen (47) ausgehärtete Klebstoff (50) den gegebenen thermischen Belastungen, insbesondere bein Autoklavieren, auf Dauer widersteht.

4. Verfahren nach einem der Ansprüche 1 bis 3, **dadurch gekennzeichnet, dass** Partikel ausgewählt werden, deren Partikelgröße im Bereich von 50 bis 150 µm liegen.

5. Verfahren nach einem der Ansprüche 1 bis 4, **dadurch gekennzeichnet, dass** elektrisch isolierende und/oder wärmeisolierende Partikel eingesetzt werden, die insbesondere aus Glas, Karbid, Kunststoff oder Keramik bestehen.

6. Verfahren nach einem der Ansprüche 1 bis 5, **dadurch gekennzeichnet, dass** Füllstoffe eingesetzt werden, die aus sphärischen, scheibenförmigen, ringförmigen, kubischen oder vielflächigen Partikeln bestehen.

7. Verfahren nach einem der Ansprüche 1 bis 4 oder 6, **dadurch gekennzeichnet, dass** der Füllstoff aus einem elektrisch leitenden und/oder wärmeleitenden, insbesondere einem metallischen Material besteht.

8. Verfahren nach Anspruch 7, **dadurch gekennzeichnet, dass** der Füllstoff aus magnetischen Partikeln besteht.

9. Verfahren nach einem der Ansprüche 1 bis 8, **dadurch gekennzeichnet, dass** der Volumenanteil der Partikel im Klebstoff, vor der Aushärtung, im Bereich von 20 bis 80 % ausgewählt wird.

10. Verfahren nach Anspruch 9, **dadurch gekennzeichnet, dass** der Volumenanteil der Partikel im Bereich von 50 bis 80 % ausgewählt wird.

11. Autoklavierbares medizinisches Instrument, bei dem zumindest ein Hohlraum (44) mit einem Klebstoffverguss, der einen Füllstoff (34) in Form von Partikeln (48) oder von Formstücken enthält, vergossen ist, **dadurch gekennzeichnet, dass** zumindest in einem Bereich (52) im Hohlraum (44) die Partikel (48) zu der dichtesten Partikelpackung, die entsprechend ihrer Geometrie möglich ist, zusammenlegen.

12. Medizinisches Instrument nach Anspruch 11, **dadurch gekennzeichnet, dass** der Füllstoff (34) im ausgehärteten Klebstoff im Bereich von 50 bis 80 Vol.-% enthalten ist.

13. Medizinisches Instrument nach einem der Ansprüche 11 und 12, **dadurch gekennzeichnet, dass** der Füllstoff als feinkörniges Pulver im Bereich von 50 bis 150 µm ausgebildet ist.

14. Medizinisches Instrument nach einem der Ansprüche 11 bis 13, **dadurch gekennzeichnet, dass** der Füllstoff aus elektrisch isolierenden und/oder wärmeisolierenden Partikeln, insbesondere aus Glas, Karbid, Kunststoff oder Keramik besteht.

15. Medizinisches Instrument nach einem der Ansprüche 11 bis 14, **dadurch gekennzeichnet, dass** der Füllstoff aus sphärischen, scheibenförmigen, ringförmigen, kubischen oder vielflächigen Partikeln besteht.

16. Medizinisches Instrument nach einem der Ansprüche 11 bis 15, **dadurch gekennzeichnet, dass** der Füllstoff aus einem elektrisch leitenden und/oder einen wärmeleitenden, insbesondere einem metallischem Material besteht.

17. Medizinisches Instrument nach Anspruch 16, **dadurch gekennzeichnet, dass** der Füllstoff aus magnetischen Partikeln besteht.

18. Medizinisches Instrument nach einem der Ansprüche 11 bis 17, **dadurch gekennzeichnet, dass** im Hohlraum ein Übergang von einer dichtesten Partikelpackung zu einem überwiegend Klebstoff enthaltenden Bereich vorhanden ist.

## Claims

1. Method for producing an autoclavable medical instrument (10), in which an adhesive (32) which contains a filler (34) as particles (48) is poured for filling a hollow space (44), **characterized in that** the adhesive (32, 50) is poured in a low-viscosity condition, and **in that** the particles (48) of the filler (34) have a density, which differs from the density of the low-viscosity adhesive, and in allowing the particles (48) of the filler (34) a sufficient period of time for packing together, at least in an area (52), to a most dense particle concentration which is possible due to its geometry, and **in that** the adhesive is then cured.

2. Method of claim 1, **characterized in that** the material of the particles (48) is chosen **in that** its thermal properties are close to the thermal properties of the constructional elements (40, 42) of the medical instrument to be poured.

3. Method of claim 1 or 2, **characterized in that** the size and the shape of the particles (48) are chosen **in that** free spaces (47) between the packed particles are of such a small size that said cured adhesive (50) within the free spaces (47) can withstand the occurring thermal stresses on a long-time basis, in particular during autoclaving.

4. Method of anyone of claims 1 through 3, **characterized in that** particles are chosen, having a size within a range of 50 µm to 150 µm.

5. Method of anyone of claims 1 through 4, **characterized in that** particles are used which are electrically insulating and/or thermally insulating, which particles are made in particular from glass, carbides, plastics or ceramics.

6. Method of anyone of claims 1 through 5, **characterized in that** fillers are used, which are made of spherical, disc-like, ring-like, cubic or polyhedral particles.

7. Method of anyone of claims 1 through 4 or claim 6, **characterized in that** the filler is made of an electric conductive and/or thermal conductive material, in particular a metallic material.

8. Method of claim 7, **characterized in that** the filler is made of magnetic particles.

9. Method of anyone of claims 1 through 8, **characterized in that** the amount per volume of said particles within the adhesive, prior to curing, is in the range of 20 to 80 %.

10. Method of claim 9, **characterized in that** the amount per volume of said particles is in the range of 50 to 80 %.

11. Autoclavable medical instrument, comprising at least one hollow space (44) containing a poured adhesive having a filler (34) as particles (48) or as shaped pieces, **characterized in that** at least in an area (52) of said hollow space (44) said particles (48) are packed together to the most dense particle concentration which is possible due to its geometry.

12. Medical instrument of claim 11, **characterized in that** the filler (34) is contained in the cured adhesive in an amount of 50 to 80 % per volume.

13. Medical instrument of claims 11 or 12, **characterized in that** the filler (34) is a fine powder in the range of 50 to 150 µm.

14. Medical instrument of anyone of claims 11 through 13, **characterized in that** the filler is made of electric isolating and/or thermal isolating particles, in particular made of glass, carbide, plastics or ceramics.

15. Medical instrument of anyone of claims 11 through 14, **characterized in that** the filler is made of spherical, disc-like, ring-like, cubic or polyhedral particles.

16. Medical instrument of anyone of claims 11 through 15, **characterized in that** the filler is made of an electric conductive and/or a thermal conductive material, in particular of a metallic material.

17. Medical instrument of claim 16, **characterized in that** the filler is made of magnetic particles.

18. Medical instrument of anyone of claims 11 through 17, **characterized in that** there is a transitional stage within said hollow space ranging from a most dense particle concentration to an area of predominantly containing adhesive.

## Revendications

1. Procédé de fabrication d'un instrument médical (10) apte au passage à l'autoclave, dans lequel une pâte adhésive (32), renfermant une substance de charge (34) revêtant la forme de particules (48), est déversée en vue de combler une cavité (44), **caractérisé par le fait que** la pâte adhésive (32, 50) est déversée sous une forme très fluide ; **par le fait que** les particules (48) de la substance de charge (34) présentent une densité différente de la densité de la pâte adhésive très fluide ; **par le fait qu'**un laps de temps suffisamment long est accordé, auxdites particules (48) de ladite substance de charge (34), pour qu'elles s'agglutinent au moins dans une région (52), jusqu'à atteindre l'agglomérat particulaire de densité maximale autorisé par leur géométrie ; et **par le fait que** le durcissement de ladite pâte adhésive s'opère après ce stade uniquement.

2. Procédé selon la revendication 1, **caractérisé par le fait que** le matériau des particules (48) est choisi de façon telle que les propriétés thermiques de ces dernières soient proches des propriétés thermiques des pièces structurelles (40, 42) de l'instrument médical destinées à être scellées.

3. Procédé selon la revendication 1 ou 2, **caractérisé par le fait que** la taille et la forme des particules (48) sont choisies pour impliquer la présence, entre les particules agglutinées, d'espaces libres (47) offrant une taille suffisamment modeste pour que la pâte adhésive (50), durcie dans lesdits espaces libres (47), résiste durablement aux contraintes thermiques considérées, en particulier lors du passage à l'autoclave.

4. Procédé selon l'une des revendications 1 à 3, **caractérisé par** le choix de particules (48) dont la taille se situe dans la plage de 50 à 150 µm.

5. Procédé selon l'une des revendications 1 à 4, **caractérisé par** l'utilisation de particules électriquement isolantes ou thermo-isolantes, consistant notamment en du verre, en un carbure, en une matière plastique ou en une céramique.

6. Procédé selon l'une des revendications 1 à 5, **caractérisé par** l'utilisation de substances de charge constituées de particules sphériques, discoïdales, annulaires, cubiques ou polyédriques.

7. Procédé selon l'une des revendications 1 à 4 ou 6, **caractérisé par le fait que** la substance de charge consiste en un matériau électriquement conducteur et/ou thermoconducteur, en particulier un matériau métallique.

8. Procédé selon la revendication 7, **caractérisé par le fait que** la substance de charge est constituée de particules magnétiques.

9. Procédé selon l'une des revendications 1 à 8, **caractérisé par le fait que** la part volumique des particules dans la pâte adhésive, préalablement au durcissement, est choisie dans la plage de 20 à 80 %.

10. Procédé selon la revendication 9, **caractérisé par le fait que** la part volumique des particules est choisie dans la plage de 50 à 80 %.

11. Instrument médical apte au passage à l'autoclave, dans lequel au moins une cavité (44) est scellée à l'aide d'une masse de pâte adhésive déversée renfermant une substance de charge (34) revêtant la forme de particules (48), ou de fragments profilés, **caractérisé par le fait que** les particules (48) sont agglutinées dans la cavité (44), au moins dans une région (52), jusqu'à atteindre l'agglomérat particulaire de densité maximale autorisé par leur géométrie.

12. Instrument médical selon la revendication 11, **caractérisé par le fait que** la substance de charge (34) est renfermée, par la pâte adhésive durcie, dans la plage de 50 à 80 % en volume.

13. Instrument médical selon l'une des revendications 11 et 12, **caractérisé par le fait que** la substance de charge est conçue comme une poudre à granulométrie fine, dans la plage de 50 à 150 µm.

14. Instrument médical selon l'une des revendications 11 à 13, **caractérisé par le fait que** la substance de charge est constituée de particules électriquement isolantes et/ou thermiquement isolantes, notamment en du verre, en un carbure, en une matière plastique ou en une céramique.

15. Instrument médical selon l'une des revendications 11 à 14, **caractérisé par le fait que** la substance de charge est constituée de particules sphériques, discoïdales, annulaires, cubiques ou polyédriques.

16. Instrument médical selon l'une des revendications 11 à 15, **caractérisé par le fait que** la substance de charge consiste en un matériau électriquement conducteur et/ou thermoconducteur, en particulier un matériau métallique.

17. Instrument médical selon la revendication 16, **caractérisé par le fait que** la substance de charge est constituée de particules magnétiques.

18. Instrument médical selon l'une des revendications 11 à 17, **caractérisé par** la présence, dans la cavité, d'une transition entre un agglomérat particulaire de densité maximale et une région renfermant de la pâte adhésive pour une part prépondérante.
